# EUROPEAN PATENT APPLICATION

(11) **EP 4 834 126 A1**
(43) Date of publication of application: **04.11.2026**
(21) Application number: 24912859.6
(22) Date of filing: 25.12.2024
(51) Int. Cl.: A23L 33/105, A23L 33/10, A61K 8/9789, A61K 36/48, A61P 43/00, A61Q 1/00, A61Q 19/08, A61K 129/00, A61K 133/00

(54) **AGENT FOR REMOVING SENESCENT CELLS AND AGENT FOR SUPPRESSING DECREASE IN MOTOR FUNCTION**

(30) Priority: 25.12.2023 JP 2023217850; 17.10.2024 JP 2024182207
(71) Applicant: Ezaki Glico Co., Ltd., Osaka-shi, Osaka 555-8502 (JP)
(72) Inventor: NAGANO,Taiki, Osaka-shi, Osaka 555-8502 (JP); KAJIURA,Hideki, Osaka-shi, Osaka 555-8502 (JP); KIMURA,Motoko, Osaka-shi, Osaka 555-8502 (JP); YAMAMOTO,So, Osaka-shi, Osaka 555-8502 (JP); TOMURA,Tomohiko, Osaka-shi, Osaka 555-8502 (JP); UJIIE,Rinako, Osaka-shi, Osaka 555-8502 (JP)
(74) Representative: Viering, Jentschura & Partner mbB Patent- und Rechtsanwälte
(86) International application number: PCT/JP2024/045803
(87) International publication number: WO 2025/142973

(57) **Abstract**

The purpose of the present invention is to provide a preparation and a composition that are effective in removal of senescent cells and can be ingested even in the field of food and drink products. Provided is an agent for removing senescent cells, the agent containing an effective amount of at least one selected from the group consisting of Albizia julibrissin flowers, Albizia julibrissin bark, and extracts of these.

## Description

### TECHNICAL FIELD

The present invention relates to an agent for removing senescent cells. More specifically, the present invention relates to an agent for removing senescent cells that selectively removes senescent cells compared with normal cells.

### BACKGROUND ART

Currently, there is an aging society mainly in developed countries, and the average life span is extended, but the difference from the healthy life span is a problem. The healthy life span is a health index proposed by the WHO (World Health Organization), and refers to a period during which a person can live independently both in mind and body and can live healthily. The difference between the healthy life span and the average life span, that is, "unhealthy period" with limitations on daily life, is said to be about 9 years for Japanese men and about 12 years for Japanese women.

From the viewpoint of countermeasures against a super-aging society, research and development of technology related to extending healthy life span has been advanced, and attention has been focused on senescence of cells or removing senescent cells. The senescent cell is a cell whose cell division has irreversibly arrested, and is present not only in the elderly but also in young people and newborns.

It has become clear that senescence of cells is induced by DNA damage due to stress such as ultraviolet rays and reactive oxygen species, shortening of telomeres, and the like. It has also been suggested that inflammatory proteins secreted by senescent cells can cause various diseases, and therefore there is a strong demand for a technique for controlling senescence of cells with which induction of senescent cells is suppressed, or generated senescent cells are removed.

For example, Patent Document 1 reports that a sodium-glucose co-transporter 2 (SGLT2) inhibitor has an action of removing senescent cells. Specifically, canagliflozin, which is a SGLT2 inhibitor, has been confirmed to have an action of removing senescent cells in an obesity model mouse, an arteriosclerosis model mouse, a progeria model mouse, and the like.

### PRIOR ART DOCUMENTS

### PATENT DOCUMENT

Patent Document 1: JP-A-2022-101541
Patent Document 2: JP-A-2010-235548

### DISCLOSURE OF THE INVENTION

### PROBLEMS TO BE SOLVED BY THE INVENTION

However, removing generated senescent cells itself is difficult, and in particular, there is almost no report on a technique capable of selectively removing senescent cells. In addition, there have still been no sufficient reports on a component that is effective for removing senescent cells and can be taken even in the field such as food and drink, using plant resources.

Therefore, an object of the present invention is to provide a preparation and a composition each of which is effective for removing senescent cells and can be taken even in the field of food and drink.

### MEANS FOR SOLVING THE PROBLEMS

In order to solve the above problems, the present inventors intensively studied and found that Albizia julibrissin, which has been eaten in the field of food, has an action of removing senescent cells, and the present invention has been completed. Furthermore, the present inventors also found that Albizia julibrissin has an action of suppressing decrease in motor function.

That is, the present invention provides the preparation and the composition described below.

### Aspect 1.

An agent for removing senescent cells, the agent including at least one selected from the group consisting of Albizia julibrissin flower, Albizia julibrissin bark, and an extract thereof, in an effective amount.

### Aspect 2.

The agent for removing senescent cells according to aspect 1, wherein the agent is labeled with at least one selected from the group consisting of "reduce senescent cells", "remove senescent cells", "reduce proportion of senescent cells", "promote reduction of senescent cells", and "suppress decrease in motor function".

### Aspect 3.

The agent for removing senescent cells according to aspect 1 or 2, wherein the removing senescent cells is selectively removing senescent cells compared with normal cells.

### Aspect 4.

The agent for removing senescent cells according to any one of aspects 1 to 3, wherein (A) 50% lethal concentration (LC50) in normal cells and (B) 50% lethal concentration in senescent cells have a ratio (A/B) of more than 1.1.

### Aspect 5.

The agent for removing senescent cells according to any one of aspects 1 to 4, wherein the agent is a food product, a cosmetic product, or a pharmaceutical product.

### Aspect 6.

An agent for suppressing decrease in motor function, the agent including at least one selected from the group consisting of Albizia julibrissin flower, Albizia julibrissin bark, and an extract thereof, in an effective amount.

### Aspect 7.

A food composition for reducing senescent cells, removing senescent cells, reducing a proportion of senescent cells, promoting reduction of senescent cells, or suppressing decrease in motor function, the food composition including at least one selected from the group consisting of Albizia julibrissin flower, Albizia julibrissin bark, and an extract thereof in an effective amount.

### Aspect 8.

Use of at least one selected from the group consisting of Albizia julibrissin flower, Albizia julibrissin bark, and an extract thereof for producing a composition for reducing senescent cells, removing senescent cells, reducing a proportion of senescent cells, promoting reduction of senescent cells, or suppressing decrease in motor function.

### Aspect 9.

The use of at least one selected from the group consisting of Albizia julibrissin flower, Albizia julibrissin bark, and an extract thereof according to aspect 8, wherein the composition is a food composition, a cosmetic composition, or a pharmaceutical composition.

Patent Document 2 does not disclose or suggest the ability of Albizia julibrissin to remove senescent cells.

### EFFECT OF THE INVENTION

According to the present invention, Albizia julibrissin, which can be taken even in the field of food and drink, is used so that senescent cells can be effectively removed. Furthermore, Albizia julibrissin is used so that decrease in motor function can be suppressed.

### BRIEF DESCRIPTION OF THE DRAWINGS

Fig. 1 is a graph showing results of ability to remove senescent cells in Test Example 1.
Fig. 2 is a graph showing results of motor function tests for a control group and an Albizia julibrissin flower extract group (0.1 mg/mL) in Test Example 2.
Fig. 3 is a graph showing results of motor function tests for a control group and an Albizia julibrissin bark extract group (0.01 mg/mL) in Test Example 2.

### MODE FOR CARRYING OUT THE INVENTION

### [Agent for removing senescent cells]

In an embodiment, the agent for removing senescent cells of the present invention contains Albizia julibrissin.

### (Albizia julibrissin)

Albizia julibrissin is a deciduous tree belonging to the Mimosoideae subfamily in the Leguminosae family. Albizia julibrissin is called nemunoki, the name of which is derived from performing a nyctinastic movement in which the small leaves close and hang down at night.

Albizia julibrissin is distributed in Honshu, Shikoku, Kyushu, and the like in Japan, and is naturally grown in Iran, Afghanistan, China, Korean Peninsula, and the like, and therefore is easily available from these regions.

Albizia julibrissin is used as a crude drug after the flower or the bark is dried in the sun, and is boiled for drinking so that it is used for improving stress or the like.

The use part of Albizia julibrissin is not particularly limited, and may be the entire plant, or may be leaf, fruit, flower, branch, trunk, root, seed, or a mixture of at least two or more thereof. In an embodiment, the use part of Albizia julibrissin preferably includes flower and/or bark, and more preferably includes flower and bark.

Albizia julibrissin may be used as raw food, or may be used after being dried (for example, freeze-dried or the like). When extraction is performed from Albizia julibrissin, it may be used after being processed into a state of crushed material or powdery material, for example, from the viewpoint of improving extraction efficiency.

For example, Albizia julibrissin can be used as crushed material or pulverized material in a chip shape, a powdery shape, a granular shape, or a paste shape, among which powder is preferably used.

The method for processing into crushed material or pulverized material is not particularly limited, and known methods may be used. Examples thereof include a process in which the plant body is crushed or pulverized by any method usually used by those skilled in the art, using equipment or tools such as a crusher, a mill, a blender, a mixer, and a stone mill.

Before being crushed or pulverized, the plant body may be dried. The method for drying process is not particularly limited, and known methods may be used. Examples thereof include freeze drying, reduced pressure drying, air blow drying, and heat drying.

When an extract of Albizia julibrissin is used, the method thereof is not particularly limited, and extraction can be performed by an ordinary extraction method used in extracting plant raw materials. Examples of the extraction method include, but are not limited to, a solvent extraction method, a steam distillation method, a squeezing method (direct, high temperature, or low temperature), and a supercritical extraction method. These extraction methods may be combined, and for example, a method in which crushing is performed and then solvent extraction is performed, or the like, may be used.

The extraction solvent is not particularly limited as long as it is an ordinary solvent used in extracting plant raw materials. The extraction solvent that can be used is, for example, an organic solvent, an aqueous solvent, or a mixture of an organic solvent and an aqueous solvent; preferably an aqueous solvent, or a mixture of an organic solvent and an aqueous solvent; and more preferably an aqueous solvent.

Examples of the organic solvent include alcohol, acetone, benzene, ester, ethyl acetate, hexane, chloroform, and diethyl ether, and alcohol is preferable. The alcohol is not particularly limited as long as it is an ordinary solvent used in extracting plant raw materials, and examples thereof include monohydric alcohols having 1 to 5 carbon atoms such as ethanol, methanol, propyl alcohol, isopropyl alcohol, and butyl alcohol; and polyhydric alcohols having 2 to 5 carbon atoms such as 1,3-butylene glycol, propylene glycol, dipropylene glycol, and glycerin.

The aqueous solvent is not particularly limited as long as it contains water, and examples thereof include water, physiological saline, and a buffer solution. Examples of the buffer solution include a phosphate buffer solution, a sodium phosphate buffer solution, a sodium carbonate buffer solution, a citrate buffer solution, a citrate phosphate buffer solution, an acetate buffer solution, and a tris buffer solution. The aqueous solvent is preferably water and/or physiological saline. The pH of the aqueous solvent is not particularly limited.

Among these extraction solvents, from the viewpoint of remarkably exhibiting the effect of the present invention, at least one selected from the group consisting of water, ethanol, 1,3-butylene glycol, and propylene glycol is preferable; water and/or ethanol is more preferable; and water or hydrous ethanol is still more preferable.

When the mixture of an organic solvent and an aqueous solvent is used as the extraction solvent, the content of the aqueous solvent contained in the entire amount of the extraction solvent is not particularly limited, and for example, 30 mass% or more, 40 mass% or more, 50 mass% or more, 60 mass% or more, 70 mass% or more, 80 mass% or more, 90 mass% or more, and 95 mass% or more.

The extraction temperature is not particularly limited, and may be, for example, -50 to 150°C, 0 to 100°C, 5 to 95°C, 10 to 95°C, 15 to 95°C, 30 to 90°C, 50 to 90°C, 70 to 90°C, and 80 to 90°C.

From the viewpoint of improving extraction efficiency, extraction is preferably performed while stirring or shaking is performed, for example. The extraction time can be appropriately determined depending on the use part such as leaf, fruit, flower, branch, trunk, root, and seed, for example. In addition, the extraction time can be appropriately determined depending on the state of Albizia julibrissin, that is, whether it is raw material or dried material; whether the state is crushed material or pulverized material; or the processing state thereof. The extraction time is usually 1 minute to 72 hours, preferably 10 minutes to 48 hours, and most preferably 30 minutes to 2 hours.

Examples of the extract from Albizia julibrissin include: an extract obtained when immersion extraction is performed with water and/or an organic solvent from flower and/or bark of Albizia julibrissin or pulverized material thereof, and the residue is filtered; a material obtained when the solvent is removed from the extract; a fine powder thereof; and a material obtained when the extract or the solvent-removed material is dissolved, dispersed, or diluted by using an appropriate solvent or the like. A commercially available product can be used therefor. As another example of the extraction method, Albizia julibrissin may be extracted after flower and/or bark of Albizia julibrissin is subjected to processing such as steaming. Leaves and branches associated with flower and/or bark of Albizia julibrissin may be removed, or may be used as they are without removing them.

The content of the plant body of Albizia julibrissin or the extract thereof is appropriately adjusted depending on the type and the amount of other components, preparation form, and the like, and is not limited, and may be, for example, 0.01 mass% or more based on the total amount of the composition, and may be 0.05 mass% or more, 0.1 mass% or more, 0.5 mass% or more, 1 mass% or more, 10 mass% or more, 20 mass% or more, 30 mass% or more, 40 mass% or more, 50 mass% or more, 60 mass% or more, 70 mass% or more, and the like. The content of the plant body of Albizia julibrissin or the extract thereof may be, for example, 100 mass% or less based on the total amount of the composition, and may be 90 mass% or less, 80 mass% or less, 70 mass% or less, 60 mass% or less, 50 mass% or less, 40 mass% or less, 30 mass% or less, 20 mass% or less, 10 mass% or less, and the like. The content of the plant body of Albizia julibrissin or the extract thereof based on the total amount of the composition may be, for example, 0.01 to 100 mass%, 0.1 to 100 mass%, 1 to 100 mass%, 5 to 100 mass%, 10 to 100 mass%, 20 to 100 mass%, 30 to 100 mass%, 40 to 100 mass%, 50 to 100 mass%, 0.01 to 90 mass%, 0.1 to 90 mass%, 1 to 90 mass%, 5 to 90 mass%, 10 to 90 mass%, 20 to 90 mass%, 30 to 90 mass%, 40 to 90 mass%, 50 to 90 mass%, 0.01 to 80 mass%, 0.1 to 80 mass%, 1 to 80 mass%, 5 to 80 mass%, 10 to 80 mass%, 20 to 80 mass%, 30 to 80 mass%, 40 to 80 mass%, 50 to 80 mass%, 0.01 to 70 mass%, 0.1 to 70 mass%, 1 to 70 mass%, 5 to 70 mass%, 10 to 70 mass%, 20 to 70 mass%, 30 to 70 mass%, 40 to 70 mass%, 50 to 70 mass%, 0.01 to 60 mass%, 0.1 to 60 mass%, 1 to 60 mass%, 5 to 60 mass%, 10 to 60 mass%, 20 to 60 mass%, 30 to 60 mass%, 40 to 60 mass%, 0.01 to 50 mass%, 0.1 to 50 mass%, 1 to 50 mass%, 5 to 50 mass%, 10 to 50 mass%, 20 to 50 mass%, 30 to 50 mass%, 0.01 to 40 mass%, 0.1 to 40 mass%, 1 to 40 mass%, 5 to 40 mass%, 10 to 40 mass%, 20 to 40 mass%, 0.01 to 30 mass%, 0.1 to 30 mass%, 1 to 30 mass%, 5 to 30 mass%, 10 to 30 mass%, 0.01 to 20 mass%, 0.1 to 20 mass%, 1 to 20 mass%, and 5 to 20 mass%.

### [Application]

The agent for removing senescent cells of the present invention is suitably used for effectively removing senescent cells, and preferably, for selectively removing senescent cells compared with normal cells. In the present specification, the senescent cell refers to a cell that has lost proliferation capability. Becoming a senescent cell is sometimes said to be a defense mechanism that when DNA is irreparably damaged by stress such as ultraviolet rays, cell division stops to prevent canceration. However, it is known that when senescent cells are not discharged and continue to remain in the living body, senescent cells secrete inflammatory proteins and the like to cause chronic inflammation. In the present specification, the normal cell, which can be compared with the senescent cell, refers to a cell that has not lost proliferation capability.

The senescent cell is identified using an increase in p16 gene and β-galactosidase activity as a marker, and it is becoming clear that when senescent cells are removed from an aged individual, aging changes thereof can be suppressed. For example, in non-alcoholic steatohepatitis (NASH), senescent cells may accumulate to cause inflammation, but it has been reported that removing senescent cells leads to improvement in inflammation.

Although there is an anti-inflammatory component for symptomatically treating inflammation caused by accumulation of senescent cells and the like, simply suppressing inflammation cannot be a fundamental solution as long as senescent cells accumulate. For the purpose of anti-aging, various components are being screened, but no sufficient reports have been made on a component that can suppress the generation of senescent cells. Furthermore, there is almost no report on a technique of distinguishing senescent cells once generated and accumulated from normal cells and selectively removing only the senescent cells.

Senescent cells accumulate not necessarily only in elderly people and patients, but may accumulate even in young people and newborns. Therefore, there is a demand not for a resolution by a pharmaceutical product, but for a technique capable of selectively removing senescent cells by a component that can be taken on a daily basis. However, there is almost no research report from such a viewpoint.

In Examples (Test Examples) described below, etoposide, which is an inducer for senescence of cells, is allowed to act on human hepatoma-derived HepG2 cells to obtain senescent cells, and then the effects of at least one selected from the group consisting of Albizia julibrissin flower, Albizia julibrissin bark, and an extract thereof are confirmed along with those of quercetin and the like, whose ability to remove senescent cells is known.

As a result, it is confirmed that at least one selected from the group consisting of Albizia julibrissin flower, Albizia julibrissin bark, and an extract thereof has an ability to remarkably and selectively remove senescent cells as compared with quercetin.

Therefore, at least one selected from the group consisting of Albizia julibrissin flower, Albizia julibrissin bark, and an extract thereof can be suitably used also for an agent for selectively removing senescent cells that effectively removes senescent cells, and preferably, can selectively remove senescent cells compared with normal cells.

The ability to selectively remove senescent cells compared with normal cells (ability to remove senescent cells) can be calculated by determining the ratio between (A) 50% lethal concentration in normal cells and (B) 50% lethal concentration in senescent cells (A/B) as in Examples described below. The ability to remove senescent cells is not limited as long as the effect of the present invention is exhibited, and for example, the ratio (A/B) is preferably more than 1.1, and may be 1.2 or more, 1.5 or more, 2 or more, 2.5 or more, 3 or more, 3.5 or more, 4 or more, 4.5 or more, 5 or more, 5.5 or more, 6 or more, 7 or more, 8 or more, and 9 or more.

### [Agent for suppressing decrease in motor function]

In an embodiment, the agent for suppressing decrease in motor function of the present invention contains Albizia julibrissin. The contents described in the embodiment of the agent for removing senescent cells are also applied in the agent for suppressing decrease in motor function.

The decrease in motor function is caused by various factors such as lack of exercise and diseases, and also gradually progresses with aging. The decrease in motor function may cause conditions or symptoms such as sarcopenia, frailty, and locomotive syndrome. In addition, the decrease in muscle strength and balance function may increase the risk of falling or bone fracture. Furthermore, the decrease in motor function may lead to lack of exercise, thereby developing or worsening a lifestyle disease.

In Examples (Test Examples) described below, the non-added control group and the Albizia julibrissin extract-added group are compared with each other to confirm the effect of suppressing decrease in motor function of at least one selected from the group consisting of Albizia julibrissin flower, Albizia julibrissin bark, and an extract thereof.

The preparation and the composition of the present invention can be used by being added to or mixed with food and drink products, cosmetic products, pharmaceutical products, quasi-pharmaceutical products, pet foods, and feeds. Alternatively, the preparation and the composition of the present invention as it is can be used as pharmaceutical products, quasi-pharmaceutical products, food and drink products, pet foods, and feeds. The preparation and the composition of the present invention can be used as food and drink products on which functionalities such as removing senescent cells and suppressing decrease in motor function are explicitly or implicitly expressed, that is, as health food products, food products labeled with claimed function, food products for patients, and special health food products. Alternatively, when the functionalities are not explicitly or implicitly expressed, they can be used as a so-called doctor's supplement recommended or presented by a doctor in the internal medicine and the orthopaedic surgery in a hospital and/or a medical office, an animal hospital, and the like. When the functionalities are explicitly expressed, they may be labeled with at least one or more selected from the group consisting of "reduce senescent cells", "remove senescent cells", "reduce proportion of senescent cells", "promote reduction of senescent cells", and "suppress decrease in motor function", but are not limited thereto. In addition, as the effects caused by the functionalities, they may be labeled with at least one or more selected from the group consisting of "improving blood liver function enzyme level", "maintain cognitive function", "maintain muscle strength", "maintain walking ability", "maintain muscle mass and muscle strength/muscle endurance necessary for living an independent daily life", "maintain agility", "decrease in fasting blood glucose level", "moderate increase in post-prandial blood glucose level", "decrease in blood neutral fat", "maintain blood vessel softness that decreases with aging", "maintain kidney filtration function", "reduce temporarily realized facial swelling and leg swelling", "reduce urination frequency", "suppressing lightheadedness", "maintain bone health", "maintain bone density", "reduce uncomfortable knee", "support flexibility and mobility of knee joint", "improve skin moisturizing ability", "maintain skin elasticity", "hair regeneration and hair restoration", "condition stomach", "improve visual function", "alleviate temporary physical and mental fatigue", "lower biological age", "improve sarcopenia", "improve frailty", "improve locomotive syndrome", "improve lifestyle disease", and "maintain balance function", but are not limited thereto.

When the functionalities are not explicitly expressed, for example, the preparation and the composition of the present invention can be used with labels such as those who are concerned about senescent cells, those who want to remove senescent cells, those who want to suppress or decrease senescent cells, those who want to reduce proportion of senescent cells, those who are concerned about numerical values of liver function and kidney function, those who feel decline in muscle strength, those who are concerned about blood glucose level, those who are concerned about bone density, those who feel uncomfortable knee joint, those who are concerned about skin conditions, those who are concerned about stomach conditions, those who are concerned about visual performance, those who feel tired, and those who are concerned about walking speed; and labels like them.

The parenteral form of the preparation and the composition of the present invention is not particularly limited, and may be an oral preparation, a parenteral preparation, and the like, and preferably an oral preparation. Examples of the oral preparation include solid or powdery preparations such as fine granule, granule, tablet, capsule, and pill; and liquid preparations such as suspension, emulsion, syrup, and extract. Examples of the parenteral preparation include injection, external preparation, and poultice.

The preparation and the composition of the present invention may consist of at least one selected from the group consisting of Albizia julibrissin flower, Albizia julibrissin bark, and an extract thereof; or may include at least one selected from the group consisting of Albizia julibrissin flower, Albizia julibrissin bark, and an extract thereof.

When the preparation and the composition of the present invention are oral preparations, other additives may be included therein. Examples thereof include excipient, binder, disintegrant, emulsifier, lubricant, flowability promoter, diluent, preservative, colorant, perfume, taste masking agent, stabilizer, humectant, antiseptic, antioxidant, and suspending agent. Examples of the additive include, but are not limited to, gelatin, sodium alginate, starch, corn starch, white sugar, lactose, glucose, mannitol, carboxymethyl cellulose, dextrin, polyvinylpyrrolidone, crystalline cellulose, soybean lecithin, sucrose, fatty acid ester, talc, magnesium stearate, polyethylene glycol, magnesium silicate, anhydrous silicic acid, and synthetic aluminum silicate.

Although the preparation and the composition of the present invention can be administered to a human, the subject to be administered may be an animal other than a human. Examples thereof include pets such as a dog, a cat, a horse, a pony, a donkey, a cow, a pig, a sheep, a goat, a rabbit, a monkey, a hamster, a guinea pig, a mouse, a rat, a ferret, a squirrel, and a bird; livestock such as a cow, a chicken, and a pig; and animals bred in ranches, zoos, and the like (a horse, a sheep, a goat, a monkey, an elephant, a lion, a cheetah, and the like).

The composition of the present invention may be a food and drink product, a cosmetic product, a pharmaceutical product, a quasi-pharmaceutical product, a pet food, a feed, and the like.

The food and drink product includes food and drink. Examples of the food and the drink include beverages (soft drinks (coffee, cocoa, juice, mineral beverage, tea beverage (green tea, black tea, oolong tea), almond beverage, sports drink, energy drink, nutritional drink, etc.), milk beverage, lactic acid bacteria beverage, yoghurt beverage, carbonated beverage, alcoholic beverage, spreads (custard cream, butter cream, peanut cream, chocolate cream, cheese cream, etc.), pastes (fruit paste, vegetable paste, sesame paste, seaweed paste, etc.), western confectionery (chocolate, donut, pie, muffin, waffle, gum, gummy, jelly, candy, cookie, cracker, biscuit, snack, cake, pudding, etc.), Japanese confectionery (candy, rice cracker, karinto, arare, dango, ohagi, daifuku, mame mochi, rice cake, bean jam, manju, castella, anmitsu, yokan, etc.), frozen confectionery (ice cream, ice candy, sherbet, etc.), retort food (curry, stew, beef bowl, chukadon, zosui, congee, miso-soup, soup, meat sauce, demiglace sauce, meat ball, hamburg steak, oden, red rice, grilled chicken, chawanmushi, etc.), instant food (instant ramen, instant soup cellophane noodle, instant soup tofu skin noodle, instant udon, instant soba, instant yakisoba, instant spaghetti, instant wonton noodle, instant shiruko, instant miso soup base, powdered soup base, source of powdered juice, hot cake mix, etc.), bottled food and canned food, jelly-like food (jelly, agar, jelly-like beverage, etc.), seasoning (soy sauce, mirin, vinegar, miso, dressing, umami seasoning, combined seasoning, sauce, mayonnaise, ketchup, furikake, tentsuyu, mentsuyu, dashi base, Chinese soup base, bouillon, barbecue sauce, cold shabu sauce, curry sauce, stew sauce, etc.), dairy products (milk, cheese, yogurt, butter, fresh cream, etc.), processed fruits (jam, marmalade, preserving in syrup, dried fruit, etc.), cereal processed food (noodle, pasta, bread, rice vermicelli., etc.), pickles (yellow pickled radish, narazuke, kimchi pickle, Fukujinzuke, pickled scallion, pickled Chinese cabbage, karashi pickle, shibazuke, asazuke, pickles, etc.), source of pickles (source of instant pickle, source of kimchi pickle, etc.), fish products (kamaboko, chikuwa, hanpen, etc.), meat products (ham, sausage, salami, bacon, etc.), dainties (sakisurume, sakitara, salted sea urchin, salted squid, salted octopus, filefish seasoned with mirin, fugu seasoned with mirin, smoked squid, salted konowata, etc.), dry foods (seasoned seaweed, etc.), daily dishes (vegetable with dressing, fried food, stir-fried food, broiled food, boiled food, vinegared dish, etc.), frozen foods (fried shrimp, croquette, spring roll, tonkatsu, shumai, gyoza, takoyaki, meat bun, bean-jam bun, etc.), baby foods (for example, rice and noodle dish, fish dish, meat dish, egg dish, dairy product dish, vegetable dish, soup, fruit, juice, rehydration beverage, tea, dessert, confectionery, sauce, and furikake); milk powders (for example, general modified milk powder, special modified milk powder, modified milk powder for pregnant woman and lactating woman); enteral nutrition food (for example, enteral nutriment, high density liquid diet, nutritional supplementary food, nutritional auxiliary food, enteral nutriment, flavor for high density liquid diet, and dessert); and other foods (nutritional auxiliary food, blender food, thickened food, food for examination, cereal food, food for sports, and emergency food).

These foods and drinks may contain, for example, food additives and food materials such as an antioxidant, a flavor, an acidulant, a colorant, an emulsifier, a preservative, a seasoning, a sweetener, a spice, a pH adjuster, a stabilizer, a vegetable oil, an animal oil, a sugar and a sugar alcohol, a vitamin, an organic acid, a fruit juice extract, a vegetable extract, cereals, beans, vegetables, meat, and fish and shellfish. The food additives and food materials are not limited thereto, and may be contained alone or in combination of two or more thereof.

The tablet can be prepared by mixing a powdery active component and a pharmaceutically acceptable carrier component (such as an excipient) and compression-molding the mixture. A confectionery tablet such as candy may be prepared by a method of injecting the mixture into a mold. The tablet may be coated with a sugar coating to form a sugar-coated tablet. Further, the tablet may be a single layer tablet or may be a laminated tablet such as a double layer tablet.

The powdery preparation such as granule may be prepared by various granulation methods (extrusion granulation method, pulverization granulation method, dry compaction granulation method, fluidized bed granulation method, rolling granulation method, high-speed stirring granulation method, and the like). A tablet can be prepared by appropriately combining the above granulation methods, tableting methods (wet tableting method and direct tableting method), and the like.

The capsule can be prepared when powdery preparations (powder, granule, and the like) are filled in a capsule (soft or hard capsule) by a conventional method.

The liquid preparation can be prepared by dissolving or dispersing each component in an aqueous medium as a carrier component (purified water, ethanol-containing purified water, and the like), filtering or sterilizing the solution as necessary, filling the solution in a predetermined container, and sterilizing the solution. The preparation form of the solid preparation is preferably a capsule or a tablet, and more preferably a soft capsule.

Soft capsules have a smooth surface and are easily swallowed, and are preferred by users. Examples of the general method for producing a soft capsule include a flat plate method, a rotary method, and a seamless method.

In production through the rotary method (punching method), the sheet-shaped capsule film sandwiches the flowing filled content, and is formed into a capsule shape along a hole of a rotating cylindrical mold. On the other hand, in production through the seamless method (dropping method), the capsule film composition and the content are simultaneously discharged from a concentric multiple nozzle, and formed into a capsule shape with no seam.

The base of the film of the soft capsule is not particularly limited, and starch, pullulan, cellulose, polyvinyl alcohol, gelatin, succinated gelatin, and the like can be used; starch, gelatin, and succinated gelatin are preferable; and gelatin and succinated gelatin are more preferable. These may be used alone or in combination of two or more thereof.

The oral intake or administration amount of Albizia julibrissin flower, Albizia julibrissin bark, or an extract thereof per day for an adult can be appropriately determined depending on state of individual, weight, sex, age, activity of material, route of intake or administration, schedule of intake or administration, preparation form, or other factors. The oral intake or administration amount of Albizia julibrissin flower, Albizia julibrissin bark, or an extract thereof per day for an adult is, for example, preferably 0.1 mg or more, more preferably 1 mg or more, still more preferably 10 mg or more, particularly preferably 20 mg or more, and most preferably 30 mg or more, and may be 100 mg or more, and 200 mg or more. The oral intake or administration amount of Albizia julibrissin flower, Albizia julibrissin bark, or an extract thereof per day for an adult is, for example, preferably 2000 mg or less, more preferably 1500 mg or less, still more preferably 1000 mg or less, and particularly preferably 500 mg or less. The oral intake or administration amount of Albizia julibrissin flower, Albizia julibrissin bark, or an extract thereof per day for an adult is, for example, preferably 0.1 to 2000 mg, more preferably 1 to 1500 mg, still more preferably 10 to 1000 mg, still more preferably 20 to 500 mg, and still more preferably 30 to 500 mg.

Note that the oral intake or administration amount per day for an adult may be, depending on the preparation form, for example as a capsule, dosed while being divided into 1 to 6 capsules, 1 to 4 capsules, 1 to 3 capsules, or 1 to 2 capsules.

The composition of the present invention can be taken or administered while being divided into one to several times per day, usually 1 to 6 times per day, 1 to 3 times per day, 1 to 2 times per day, or at any period and interval, and one time per day is preferable.

### EXAMPLES

Next, the present invention will be specifically described with reference to Examples, but the present invention is not limited to the following Examples.

### [Preparation of a test sample]

### (Example 1. Ethanol extract 1)

For extraction, 30 mL of 99.5% ethanol was added to 3.0 g of dry powder of Albizia julibrissin flower, and stirred at room temperature for 30 minutes. Thereafter, the residue was removed by a conventional method, and the resulting extract was evaporated to dryness to obtain 0.1326 g of a dried product.

### (Example 2. Ethanol extract 2)

For extraction, 30 mL of 99.5% ethanol was added to 3.0 g of dry powder of Albizia julibrissin bark, and stirred at room temperature for 30 minutes. Thereafter, the residue was removed by a conventional method, and the resulting extract was evaporated to dryness to obtain 0.1551 g of a dried product.

### (Example 3. Hot water extract 1)

For extraction, 27 mL of water was added to 3.0 g of dry powder of Albizia julibrissin flower, and the mixture was stirred at 90°C for 1 hour. Thereafter, the residue was removed by a conventional method, and the resulting extract was evaporated to dryness to obtain 0.7323 g of a dried product.

### (Example 4. Hot water extract 2)

For extraction, 27 mL of water was added to 3.0 g of dry powder of Albizia julibrissin bark, and the mixture was stirred at 90°C for 1 hour. Thereafter, the residue was removed by a conventional method, and the resulting extract was evaporated to dryness to obtain 0.1851 g of a dried product.

### (Example 5. Alkaline extract)

After 25 mL of water was added to 3.0 g of dried powder of Albizia julibrissin flowers, the mixture was adjusted to pH 9.0 (±0.1) using a 0.5 mol/L sodium hydroxide solution, and the total mass was adjusted to 33 g with water. The mixture was then stirred at 70°C for 1 hour for extraction. The mixture was adjusted to pH 7.0 using 0.5 mol/L hydrochloric acid, then the residue was removed by a conventional method, and then the resulting extract was evaporated to dryness to obtain 0.5802 g of a dried product.

### [Test Example 1: Test for confirming ability to remove senescent cells]

### (1) Preparation of senescent cells

On a 12 well plate, 10,000 human hepatoma-derived HepG2 cells were seeded, and treated with 10µM (containing 0.1% DMSO) of etoposide (Sigma-Aldrich) for 2 days to induce senescence of cells. In order to confirm induction of senescence of cells, the cells were treated with 1 µM SPiDER-βGal (Dojindo), and observed for fluorescence signals by using All-in-One Fluorescence Microscope BZ-9000 (Keyence) with a GFP filter (excitation wavelength: 470/40 nm, absorption wavelength: 525/50 nm). As a result, conversion of SPiDER-βGal into a fluorescent substance, which is caused by the β-galactosidase activity of senescent cells, was observed in the cells treated with etoposide.

### (2) Evaluation of activity to remove senescent cells

The ratio between 50% lethal concentration (Lethal Concentration 50, hereinafter referred to as "LC50") in normal (non-senescent) cells and LC50 in senescent cells was confirmed. LC50 is the concentration required to kill half of the cells in the plate.

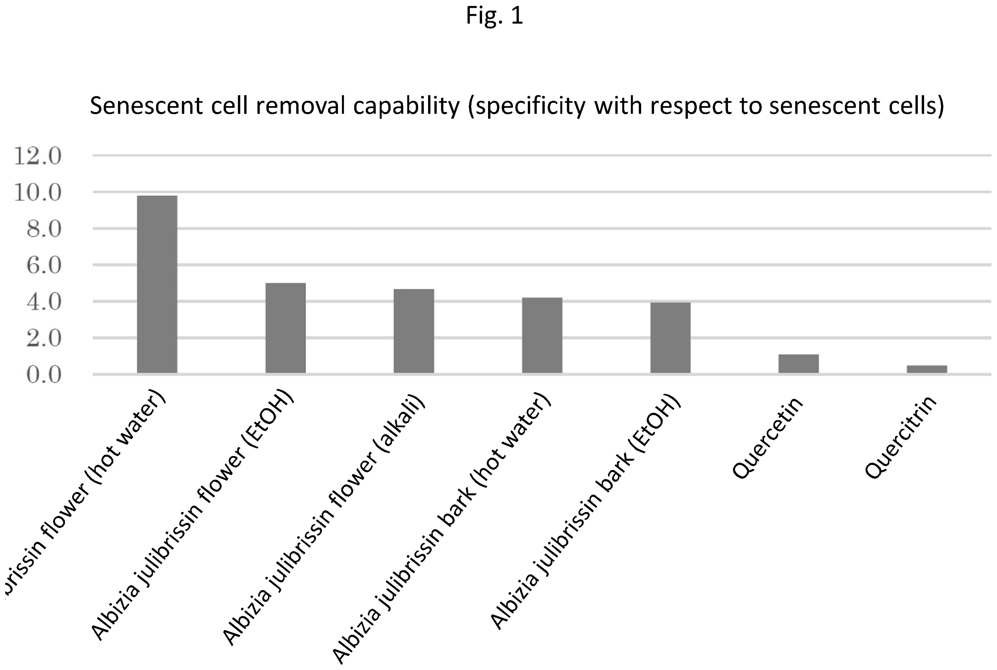

On a 96 well plate, 8,000 HepG2 cells were seeded, and treated with 10µM (containing 0.1% DMSO) of etoposide for 2 days to induce senescence of cells. Thereafter, in an RPMI medium containing 0.5% fetal bovine serum (not containing HEPES) (ThermoFisher), the test preparation prepared above was added so that 0.179 to 40 µg/mL of the extract of Albizia julibrissin flower and Albizia julibrissin bark was contained, to treat the cells, and the number of cells after two days was measured with EnVision (PerkinElmer) in terms of amount of luminescence using CellTiter-Glo (Promega). Based on the results of measurement values of the treatment concentrations, LC50 was calculated with GraphPad Prism9 (GraphPad Software). Based on the equation below, the results of determining the ability to remove senescent cells are shown in Fig. 1.

### How to determine ability to remove senescent cells

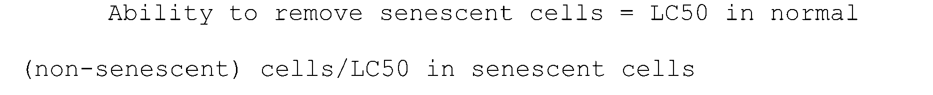

Ability to remove senescent cells = LC50 in normal (non-senescent) cells/LC50 in senescent cells

As shown in Fig. 1, the ability to remove senescent cells, which is the ability to induce cell death specific to senescent cells, was determined by the above equation. As a result, in the Albizia julibrissin flower extracts, the obtained values were 9.8 (hot water extraction), 5.0 (ethanol extract), and 4.7 (alkaline extraction); and in the Albizia julibrissin bark extracts, the obtained values were 4.2 (hot water extraction) and 3.9 (ethanol extract). Since the ability to remove senescent cells of quercetin, whose action of removing senescent cells is known, was studied under the same conditions, and the result thereof was 1.1, it was confirmed that the Albizia julibrissin flower extract and the Albizia julibrissin bark extract have higher ability to remove senescent cells than known quercetin.

### [Test Example 2: Motor function test]

### (1) Breeding fruit fly

Wild-type fruit flies (strain W¹¹¹⁸) were used for the experiment. Male fruit flies after emergence were bred in an environment of a temperature of 25 ± 3°C, a humidity of 70 ± 20%, and a 12-hour light-dark cycle. For the control group, a normal bait (4.4 w/w% of corn flour, 5 w/w% of dry yeast Y4, 0.44 w/w% of powdered agar, 5.6 w/w% of glucose, 0.44 w/w% of propionic acid, and 0.41 w/w% of bokinin); for the Albizia julibrissin flower extract group (0.1 mg/mL), a bait to which an Albizia julibrissin flower extract prepared in the same manner as in Example 3. Hot water extract 1 was added in a final concentration of 0.1 mg/mL; and for the Albizia julibrissin bark extract group (0.01 mg/mL), a bait to which an Albizia julibrissin bark extract prepared in the same manner as in Example 4. Hot water extract 2 was added in a final concentration of 0.01 mg/mL was provided at the bottom of a vial (diameter 22 mm, height 96 mm), and was fed free. The fruit flies were transferred to a vial in which a new bait was placed once a week.

### (2) Evaluation of motor function

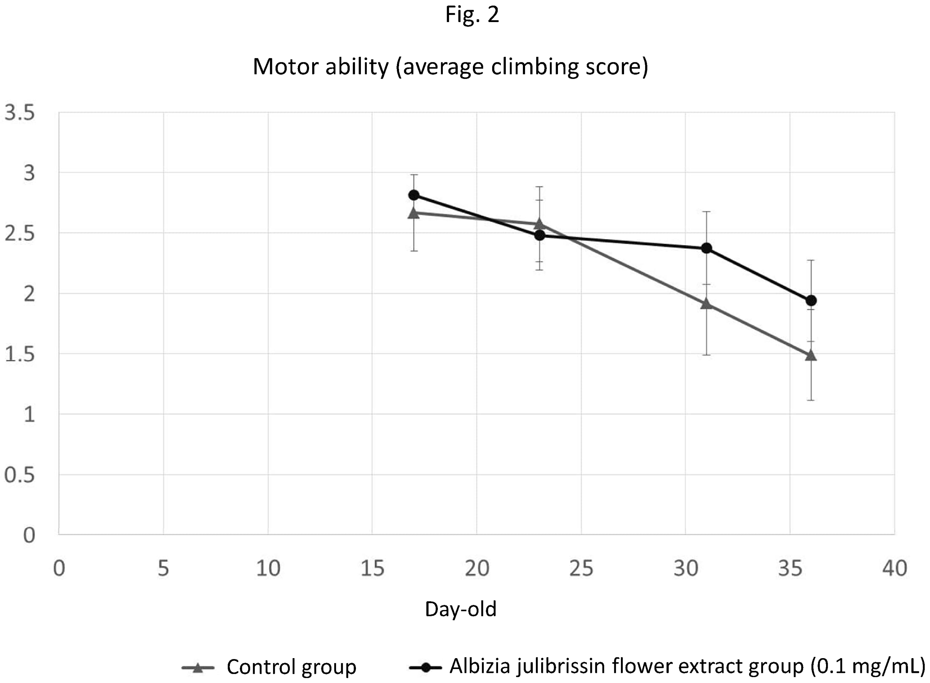

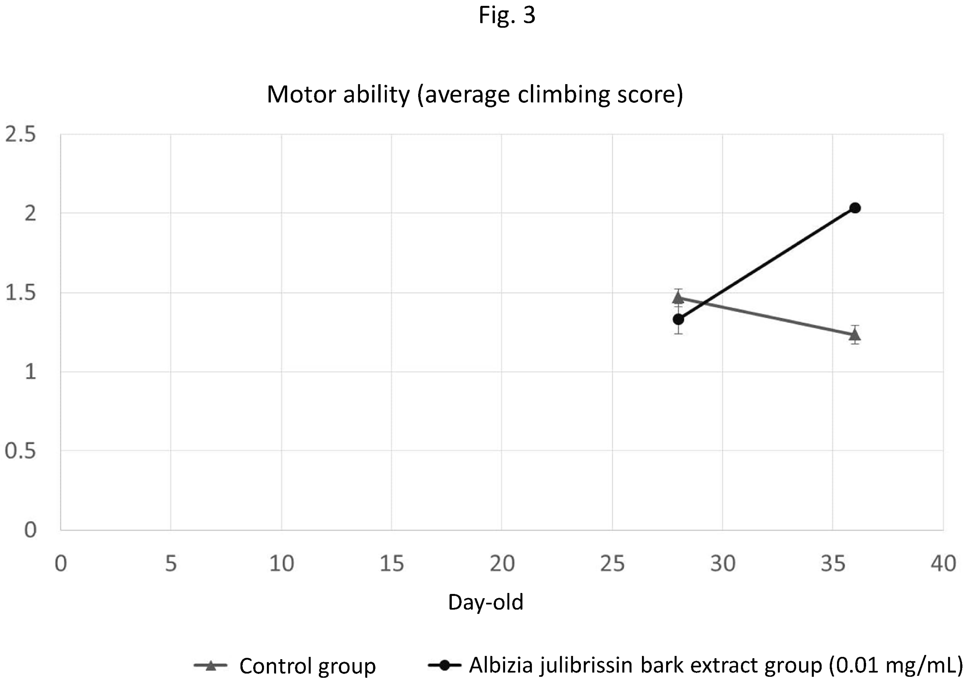

As for the motor function of fruit flies, a climbing assay was performed to evaluate negative geotaxis. The climbing assay was performed under the following conditions. Ten fruit flies per vial were transferred to an empty vial in which no bait was contained, and the vial was tapped to drop the fruit flies to the bottom, and then video photographing was performed on the fruit flies climbing the wall of the vial. Ten seconds after the fruit flies were dropped to the bottom, the number of flies was counted for each of the upper 1/3 range, the middle 1/3 range, or the lower 1/3 range of the vial. For one fly, a fly in the upper part of the vial was 3 point, a fly in the middle part was 2 point, and a fly in the lower part was 1 point, and the total score was calculated for each group, and the total score was divided by the number of flies to calculate the average climbing score. The average climbing score was determined for each day-old, and the results were shown in Tables 1 and 2, and Figs. 2 and 3, respectively.

### (3) Evaluation of improvement in motor function by Albizia julibrissin flower extract

**[Table 1]**

| | Motor ability (average climbing score) | |
|---|---|---|
| | Control group | Albizia julibrissin flower extract group (0.1 mg/mL) |
| 17 day-old | 2.67 | 2.81 |
| 23 day-old | 2.57 | 2.48 |
| 31 day-old | 1.92 | 2.37 |
| 36 day-old | 1.49 | 1.94 |

As shown in Table 1 and Fig. 2, the average climbing score, showing the motor ability of the fruit flies, was determined. As a result, in the Albizia julibrissin flower extract administration group, a value of 1.94 was obtained for 36-day-old. In the control group, the score for the same day-old was 1.49. Therefore, it was confirmed that the Albizia julibrissin flower extract has the ability to suppress decrease in motor function.

### (4) Evaluation of improvement in motor function by Albizia julibrissin bark extract

**[Table 2]**

| | Motor ability (average climbing score) | |
|---|---|---|
| | Control group | Albizia julibrissin bark extract group (0.01 mg/mL) |
| 28 day-old | 1.47 | 1.33 |
| 36 day-old | 1.23 | 2.04 |

As shown in Table 2 and Fig. 3, the average climbing score, showing the motor ability of the fruit flies, was determined. As a result, in the Albizia julibrissin bark extract administration group, a value of 2.04 was obtained for 36-day-old. In the control group, the score for the same day-old was 1.23. Therefore, it was confirmed that the Albizia julibrissin bark extract has the ability to suppress decrease in motor function.

## Claims

1. An agent for removing senescent cells, the agent comprising at least one selected from the group consisting of Albizia julibrissin flower, Albizia julibrissin bark, and an extract thereof, in an effective amount.

2. The agent for removing senescent cells according to claim 1, wherein the agent is labeled with at least one selected from the group consisting of "reduce senescent cells", "remove senescent cells", "reduce proportion of senescent cells", "promote reduction of senescent cells", and "suppress decrease in motor function".

3. The agent for removing senescent cells according to claim 1, wherein the removing senescent cells is selectively removing senescent cells compared with normal cells.

4. The agent for removing senescent cells according to claim 1, wherein (A) 50% lethal concentration (LC50) in normal cells and (B) 50% lethal concentration in senescent cells have a ratio (A/B) of more than 1.1.

5. The agent for removing senescent cells according to claim 1, wherein the agent is a food product, a cosmetic product, or a pharmaceutical product.

6. An agent for suppressing decrease in motor function, the agent comprising at least one selected from the group consisting of Albizia julibrissin flower, Albizia julibrissin bark, and an extract thereof, in an effective amount.
